# EUROPEAN PATENT APPLICATION

(11) **EP 3 791 784 A1**
(43) Date of publication of application: **17.03.2021**
(21) Application number: 19800659.5
(22) Date of filing: 25.04.2019
(51) Int. Cl.: A61B 5/0444, A61B 5/0452

(54) **FETAL ELECTROCARDIOGRAPHIC SIGNAL PROCESSING METHOD AND FETAL ELECTROCARDIOGRAPHIC SIGNAL PROCESSING DEVICE**

(30) Priority: 10.05.2018 JP 2018091128
(71) Applicant: Atom Medical Corporation, Bunkyo-ku Tokyo 113-0033 (JP)
(72) Inventor: SUDO, Kazuhiko, Saitama City, Saitama 338-0835 (JP); ODAGIRI, Naoko, Saitama City, Saitama 338-0835 (JP); OOWADA, Kazunari, Saitama City, Saitama 338-0835 (JP); SONE, Yoshikatsu, Saitama City, Saitama 338-0835 (JP); KUBO, Masayuki, Saitama City, Saitama 338-0835 (JP); MATSUBARA, Ichiro, Tokyo 113-0033 (JP)
(74) Representative: DREISS Patentanwälte PartG mbB
(86) International application number: PCT/JP2019/017759
(87) International publication number: WO 2019/216251

(57) **Abstract**

Provided are a fetal electrocardiographic signal processing method and a fetal electrocardiographic signal processing device that can appropriately select a signal reliably including a fetal electrocardiographic component from a plurality of signals separated by independent component analysis with reference.

The fetal electrocardiographic signal processing method according to the invention includes: a fetal feature display signal extraction step of separating separation signals for a plurality of channels from biological signals of the plurality of channels acquired from a pregnant mother, using independent component analysis with reference, and removing noise from the separation signal for each channel to extract a fetal feature display signal; and a maternal electrocardiographic signal removal step of removing the fetal feature display signal at a timing when an electrocardiographic signal of the mother is likely to appear from the fetal feature display signals to obtain fetal feature signals including a large number of fetal electrocardiographic signals.

## Description

### BACKGROUND OF THE INVENTION

### Technical Field

The present invention relates to a fetal electrocardiographic signal processing method and a fetal electrocardiographic signal processing device that can appropriately acquire an electrocardiographic signal of a fetus in a mother's body. The present application claims priority based on Japanese Patent Application No. 2018-91128 filed on May 10, 2018, the contents of which are incorporated herein by reference.

### Background Art

In recent years, the number of devices which acquire biological signals, such as breathing, a pulse, a heart sound, body temperature, and sweating, from a noncontact-type electrode (for example, a piezoelectric element, a millimeter-wave radar, or an optical sensor) or a contact-type electrode (for example, a conductive fiber, a wristwatch-type optical sensor, or a small wearable device) having a different shape and material from those according to the related art has increased. It is necessary to perform a filtering process on the biological signal in order to attenuate noise. However, it is difficult to extract a target signal using only a frequency filter, such as a bandpass filter, and it is necessary to perform a noise removal process corresponding to each signal.

An electrocardiographic signal processing method has been known which separates and extracts a fetal electrocardiographic signal from biological signals including a maternal electrocardiographic signal and a fetal electrocardiographic signal among the biological signals (see Patent Document 1).

The electrocardiographic signal processing method described in Patent Document 1 generates a reference signal for separating and extracting a fetal electrocardiographic signal of a designated induction form on the basis of a heartbeat cycle signal input from a detector that detects a heartbeat cycle of a fetus, and separates and extracts the fetal electrocardiographic signal of the designated induction form from biological signals including maternal electrocardiographic signals and fetal electrocardiographic signals of a plurality of channels acquired from a pregnant mother using independent component analysis with reference.

Then, a main component of the maternal electrocardiographic signal from a channel of an electrode attached to the chest of the mother's body is removed from the biological signal of a channel of another electrode to separate the fetal electrocardiographic signal. Therefore, a fetal electrocardiographic component and the other components are separated from the input signals of a plurality of channels separated by the independent component analysis with reference to generate a signal including the fetal electrocardiographic component.

### Citation List

### Patent Literature

Patent Document 1: JP-A-2006-204759

### SUMMARY OF INVENTION

### Technical Problem

However, in the configuration disclosed in Patent Document 1, in the measurement of the fetal electrocardiographic signal, unlike the measurement of the electrocardiographic signal of an adult, it is difficult to directly attach the electrode to the fetus. Therefore, it is difficult to designate which part the electrode is attached to and the acquired signal is not necessarily detected in the desired direction. Further, in a case in which a sufficiently large amplitude is not obtained due to the direction of the R-wave of the extracted fetal electrocardiographic component or the strength of a spectrum, the fetal electrocardiographic component is likely to be affected by noise and it is difficult to recognize the fetal electrocardiographic component. In this case, an erroneous signal is likely to be selected as a signal including the fetal electrocardiographic component after analysis.

The invention has been made in view of the above-mentioned problems and an object of the invention is to provide a fetal electrocardiographic signal processing method and a fetal electrocardiographic signal processing device that can appropriately select a signal reliably including a fetal electrocardiographic component from a plurality of signals separated by independent component analysis with reference.

### Solution to Problem

A fetal electrocardiographic signal processing method according to the invention includes: a fetal feature display signal extraction step of separating a separation signal for each channel from biological signals of a plurality of channels acquired from a pregnant mother, using independent component analysis with reference, and removing noise from each separation signal to extract a fetal feature display signal for each channel; and a maternal electrocardiographic signal removal step of removing the fetal feature display signal at a timing when an electrocardiographic signal of the mother is likely to appear from the fetal feature display signals to obtain fetal feature signals including a large number of fetal electrocardiographic signals.

Here, in the separation signal separated by the independent component analysis with reference, noise or the maternal electrocardiographic signal is removed to some extent, but is not completely removed. In addition, in a case in which a sufficiently large amplitude is not obtained due to the direction of the R-wave of the fetal electrocardiographic component or the strength of a spectrum, the fetal electrocardiographic component is likely to be affected by noise and it is difficult to recognize the fetal electrocardiographic component. In this case, an erroneous signal is likely to be selected as a signal including the fetal electrocardiographic component after analysis.

In contrast, in the invention, noise is removed from the separation signals separated by the independent component analysis with reference to extract the fetal feature display signals, and a signal at the timing when the maternal electrocardiographic signal is likely to be included is removed from the extracted fetal feature display signals, which makes it possible to reliably remove noise and the maternal electrocardiographic signal. Therefore, it possible to obtain the fetal feature signal including a large number of fetal electrocardiographic signals, and the fetal feature signals of each channel are compared to appropriately select a separation signal reliably including the fetal electrocardiographic component.

As a preferred aspect of the fetal electrocardiographic signal processing method according to the invention, the fetal feature display signal extraction step may include: a complex signal generation step of generating a plurality of complex signals including an imaginary part signal obtained by shifting a phase of the separation signal for each channel; and a specific frequency band component extraction step for extracting a specific frequency band component of each complex signal for each channel. The fetal feature display signal obtained by removing noise from the specific frequency band component may be acquired.

It is desirable that the specific frequency band is equal to or greater than 10 Hz and equal to or less than 40 Hz. This is because the fetal R-wave component included in the separation signal separated by the independent component analysis with reference mainly includes a frequency of 10 to 40 Hz. That is, in the specific frequency band component extraction step, a frequency band component including a large number of fetal R-wave components is extracted.

In the above-mentioned aspect, the phases of the separation signals for a plurality of channels are shifted to generate a plurality of complex signals including an imaginary part signal. Therefore, it is possible to increase the number of samples for extracting the fetal feature signal and to obtain a plurality of fetal feature signals for each channel. Therefore, since the number of fetal feature signals to be compared is large, it is possible to appropriately select a signal reliably including the fetal electrocardiographic component from the separation signals.

As a preferred aspect, the fetal electrocardiographic signal processing method according to the invention may further include: a data compression step of compressing a plurality of samples forming the fetal feature signal to generate compressed data for each channel; a signal selection step of calculating a standard deviation of the compressed data based on each complex signal for each channel, selecting compressed data with a second largest standard deviation, and sorting the selected compressed data of each channel in descending order of the standard deviation; and an image generation step of sorting and displaying the separation signals corresponding to the compressed data sorted in descending order of the standard deviation. In the data compression step, the plurality of samples may be allocated in units of a predetermined number of samples, a maximum value of the predetermined number of allocated samples may be extracted, and the predetermined number of samples may be set as one sample including the maximum value.

Here, in the analysis process, an increase in the number of samples leads to an increase in processing time. Therefore, it is desirable to delete redundant data as much as possible and to perform the analysis, in order to reduce the processing time.

In contrast, in the above-mentioned aspect, it is possible to select a separation signal including a large number of fetal electrocardiographic components, using the compressed data generated by allocating a plurality of samples in units of a predetermined number of samples from the fetal feature signal and setting the predetermined number of samples as one sample including the maximum value of the predetermined number of samples. Therefore, it is possible to reduce the processing time.

In addition, since the compressed data having the second largest standard deviation is selected, it is possible to more reliably select a separation signal including a large number of fetal electrocardiographic components. Further, since the separation signals corresponding to the compressed data sorted in descending order of the standard deviation are sorted and displayed, it is possible to reliably recognize the separation signal including a large number of fetal electrocardiographic components.

A fetal electrocardiographic signal processing device according to the invention includes: a fetal feature display signal extraction unit that separates a separation signal for each channel from biological signals of a plurality of channels acquired from a pregnant mother, using independent component analysis with reference, and removes noise from each separation signal to extract a fetal feature display signal for each channel; and a maternal electrocardiographic signal removal unit that removes the fetal feature display signal at a timing when an electrocardiographic signal of the mother is likely to appear from the fetal feature display signals to obtain fetal feature signals including a large number of fetal electrocardiographic signals.

As a preferred aspect of the fetal electrocardiographic signal processing device according to the invention, the fetal feature display signal extraction unit may include: a complex signal generation unit that generates a plurality of complex signals including an imaginary part signal obtained by shifting a phase of the separation signal for each channel; and a specific frequency band component extraction unit that extracts a specific frequency band component of each complex signal for each channel. The fetal feature display signal extraction unit may acquire the fetal feature display signal obtained by removing the noise from the specific frequency band component.

As a preferred aspect, the fetal electrocardiographic signal processing device according to the invention may further include: a data compression unit that compresses a plurality of samples forming the fetal feature signal to generate compressed data for each channel; a signal selection unit that calculates a standard deviation of the compressed data based on each complex signal for each channel, selects compressed data with a second largest standard deviation, and sorts the selected compressed data of each channel in descending order of the standard deviation; and an image generation unit that sorts and displays the separation signals corresponding to the compressed data sorted in descending order of the standard deviation. The data compression unit may allocate a plurality of samples in units of a predetermined number of samples, extract a maximum value of the predetermined number of allocated samples, and set the predetermined number of samples as one sample including the maximum value.

### Advantageous Effects of Invention

According to the invention, it is possible to appropriately select a signal that reliably includes a fetal electrocardiographic component from a plurality of signals separated by the independent component analysis with reference.

### BRIEF DESCRIPTION OF DRAWINGS

FIG. 1 is a flowchart illustrating a procedure of fetal electrocardiographic signal processing (channel re-sorting process) according to an embodiment of the invention.
FIG. 2 is a diagram schematically illustrating an overall configuration of a fetal electrocardiographic signal processing device in the embodiment.
FIG. 3 is a block diagram illustrating a configuration of a channel re-sorting unit in the embodiment.
FIG. 4 is a diagram illustrating a plurality of phase signals for each channel in the embodiment.
FIG. 5 is a diagram illustrating a complex signal in the embodiment. FIG. 5(a) illustrates waveform data indicating a real part of a separation signal separated by independent component analysis with reference, FIG. 5(b) illustrates waveform data indicating an imaginary part of the separation signal separated by the independent component analysis with reference in addition to the real part, and FIG. 5(c) illustrates waveform data indicating an example of all of four complex signals.
FIG. 6 illustrates data indicating an example of a fetal feature display signal in the embodiment. FIG. 6(a) illustrates data before a fetal feature display signal at the timing when a maternal electrocardiographic signal is likely to appear is removed and FIG. 6(B) illustrates data indicating a range in which the fetal feature display signal at the timing when the maternal electrocardiographic signal is likely to appear is removed.
FIG. 7 is a diagram illustrating data after the fetal feature display signal at the timing when the maternal electrocardiographic signal is likely to appear is removed in the embodiment. FIG. 7(a) illustrates data before compression and FIG. 7(b) illustrates data after compression.
FIG. 8 is a diagram illustrating an example of an image displayed on a monitor in the embodiment before channel re-sorting.
FIG. 9 is a diagram illustrating an example of an image displayed on the monitor in the embodiment after channel re-sorting.

### DESCRIPTION OF EMBODIMENTS

Hereinafter, embodiments of a medical apparatus including a fetal electrocardiographic signal processing device and a fetal electrocardiographic signal processing method according to the invention will be described with reference to the drawings.

### [Schematic Configuration of Medical Apparatus]

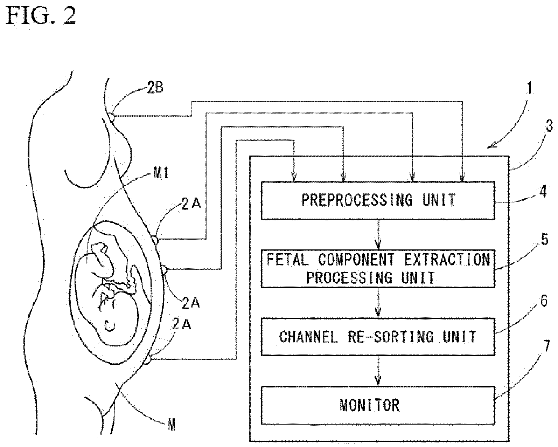

A medical apparatus 1 according to this embodiment is a so-called fetal monitoring apparatus and includes a plurality of electrodes 2A and 2B that are attached to a pregnant mother M and an apparatus main body 3 to which biological signals including electrocardiographic signals of the mother M and electrocardiographic signals of a fetus M1 acquired by the plurality of electrodes 2A and 2B are transmitted as illustrated in FIG. 2. For example, in this embodiment, the medical apparatus includes 11 electrodes 2A (hereinafter, referred to as fetal electrodes 2A) for acquiring the electrocardiographic signals of the fetus M1 and one electrode 2B (hereinafter, referred to as a maternal electrode 2B) for acquiring the electrocardiographic signal of the mother M. A data communication path for acquiring the electrocardiographic signals of the fetus M1 is configured by 11 channels corresponding to the 11 fetal electrodes 2A.

The apparatus main body 3 includes: a preprocessing unit 4 that receives biological signals from a plurality of (11 in this embodiment) channels and performs preprocessing; a fetal component extraction processing unit 5 that extracts (separates) fetal components (signals) from the biological signals subjected to the preprocessing; a channel re-sorting unit 6 that processes the signals extracted by the fetal component extraction processing unit 5 and appropriately selects and re-sorts signals including a large number of fetal electrocardiographic components; and a monitor 7 that displays waveform data of the signals (signals corresponding to a fetal electrocardiogram) re-sorted by the channel re-sorting unit 6.

Among these units, the fetal component extraction processing unit 5 performs the method disclosed in the above-mentioned PTL 1. Specifically, the fetal component extraction processing unit 5 generates a reference signal for separating and extracting a fetal electrocardiographic signal of a designated induction form on the basis of a heartbeat cycle signal input from a detector that detects the heartbeat cycle of the fetus M1, and separates and extracts the fetal electrocardiographic signals of the designated induction form from biological signals including maternal electrocardiographic signals and fetal electrocardiographic signals of each channel acquired from the pregnant mother M on the basis of the reference signal, using independent component analysis with reference.

Then, the fetal electrocardiographic signal is separated by removing a main component of the maternal electrocardiographic signal from the channel of the electrode attached to the chest of the mother M from the biological signals of the channels of the other electrodes.

This embodiment is characterized in that the channel re-sorting unit 6 can process separation signals (fetal electrocardiographic signals) separated by the fetal component extraction processing unit 5 to extract fetal feature display signals, remove a fetal feature display signal at the timing when the electrocardiographic signal of the mother M is likely to appear from the extracted fetal feature display signals to obtain a fetal feature signal including a large number of electrocardiographic signals of the fetus M1, and select a separation signal (fetal electrocardiographic signal) reliably including the fetal electrocardiographic component from the separation signals separated by the fetal component extraction processing unit 5 on the basis of the fetal feature signal. This will be described in detail below.

### [Configuration of Plurality of Electrodes]

The plurality of electrodes 2A and 2B are so-called ultrasonic sensors and are attached to the chest or abdomen of the mother M. For example, one maternal electrode 2B is attached to the chest of the mother M in order to detect the electrocardiographic signal of the mother M and 11 fetal electrodes 2 are attached to the abdomen of the mother M.

The electrodes 2A and 2B detect biological signals including, for example, the electrocardiographic signal of the mother M from the heart of the pregnant mother M, the electrocardiographic signals of the fetus M1 from the heart of the fetus M1, and noise. Then, the biological signals detected by each of the electrodes 2A and 2B are transmitted to the preprocessing unit 4 of the apparatus main body 3 through a cable (not illustrated). The detection of the biological signals by each of the electrodes 2A and 2B is performed, for example, for about 20 minutes.

### [Configuration of Preprocessing Unit]

The preprocessing unit 4 converts the biological signals detected from each of the electrodes 2A and 2B into digital signals in order to analyze the biological signals. Specifically, the preprocessing unit 4 measures the biological signals input from the electrodes 2A and 2B and amplifies the electrocardiographic signals of the fetus M1 included in the biological signals. In addition, the preprocessing unit 4 converts analog data (biological signal) into digital data, accumulates data for each block that can be calculated online, and outputs the data to the fetal component extraction processing unit 5.

### [Configuration of Fetal Component Extraction Processing Unit]

As described above, the fetal component extraction processing unit 5 separates the fetal electrocardiographic components (separation signals) for each channel from the biological signals of each channel acquired from the preprocessing unit 4, using the independent component analysis with reference, and outputs the separation signals for each channel to the channel re-sorting unit 6.

Specifically, the fetal component extraction processing unit 5 separates the fetal electrocardiographic components for each channel (separation signals) from the biological signals of the 11 channels acquired from the preprocessing unit 4. Then, the fetal component extraction processing unit 5 transmits the fetal electrocardiographic components (separation signals) of 15000 samples (samples corresponding to 15 seconds since 1000 samples per second are transmitted) for each channel to the channel re-sorting unit 6.

### [Configuration of Channel Re-sorting Unit]

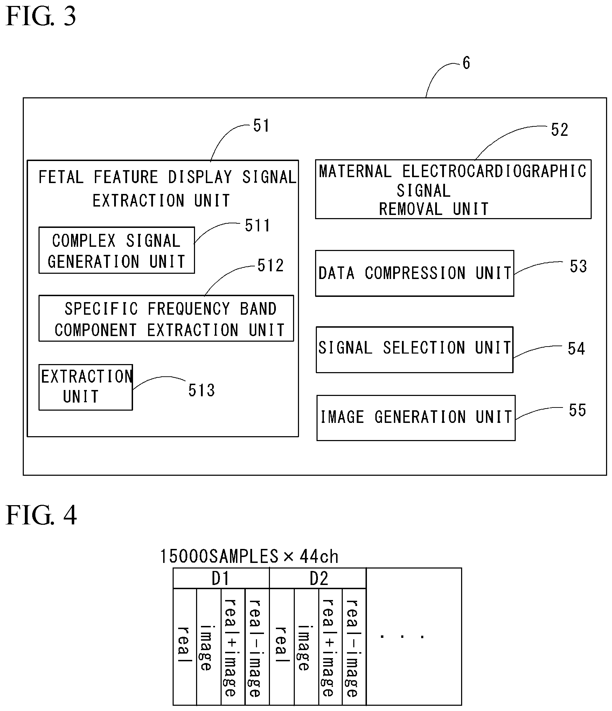

As illustrated in FIG. 3, the channel re-sorting unit 6 includes: a fetal feature display signal extraction unit 51 that removes noise from the separation signals acquired from the fetal component extraction processing unit 5 to extract fetal feature display signals; a maternal electrocardiographic signal removal unit 52 that removes a signal at the timing when the electrocardiographic signal of the mother M is likely to appear from the fetal feature display signals extracted by the fetal feature display signal extraction unit to obtain fetal feature signals including a large number of electrocardiographic signals of the fetus M1; a data compression unit 53 that compresses a plurality of samples forming the fetal feature signal for each channel to generate compressed data; a signal selection unit 54 that calculates a standard deviation of the compressed data based on each complex signal for each channel, extracts compressed data having the second largest standard deviation, and sorts the extracted compressed data of each channel in descending order of the standard deviation; and an image generation unit 55 that sorts and displays the separation signals corresponding to the compressed data sorted in descending order of the standard deviation.

Among these units, the fetal feature display signal extraction unit 51 includes: a complex signal generation unit 511 that shifts the phase of the separation signal for each channel to generate a plurality of complex signals including an imaginary part signal; a specific frequency band component extraction unit 512 that extracts a specific frequency band component of the plurality of complex signals for each channel; and an extraction unit 513 that converts the specific frequency band component into an absolute value, calculates a standard deviation σ, standardizes the specific frequency band component, and extracts the fetal feature display signal.

### [Configuration of Complex Signal Generation Unit]

The complex signal generation unit 511 shifts the phase of the signal separated by the independent component analysis with reference for each channel to generate a plurality of complex signals including an imaginary part signal. Specifically, the complex signal generation unit 511 performs a process of performing the Hilbert transform on the separation signals of each of channels D1 to D11 to delay the phases of the signals by 90°, thereby generating an imaginary part of each of the separation signals ("image" illustrated in FIG. 4).

Further, the complex signal generation unit 511 generates a complex signal ("real ± image" illustrated in FIG. 4) including the imaginary part signal of the separation signal. The complex signal including the imaginary part signal of the separation signal consists of a signal obtained by delaying the phase of the separation signal by 45° and a signal obtained by advancing the phase of the separation signal by 45°. Therefore, in this embodiment, four complex signals including the imaginary part signal are generated for each of the channels D1 to D11. That is, the complex signal generation unit 511 quadruples each of the separation signals of the channels D1 to D11 to generate four complex signals. Therefore, complex signals of 44ch × 15000 samples are generated.

The reason why four complex signals are generated is as follows. In an imaginary part of the separation signal, an R-wave larger than that in a real part of the separation signal is likely to appear and the imaginary part is used as a material for determination when the features of the fetus M1 are extracted. That is, the object of this process is not to calculate the height or shape of the peak of the separation signal, but is to make the fetal electrocardiographic component conspicuous and to extract and select a separation signal including a large number of fetal electrocardiographic components. Therefore, four complex signals are generated.

In this embodiment, the number of complex signals including the imaginary part signal is quadrupled. However, the invention is not limited thereto. For example, the number of complex signals may be doubled or the real part of the separation signal may not be used. Further, the Hilbert transform is used to generate the imaginary part of the separation signal. However, the invention is not limited thereto. The imaginary part of the separation signal may be generated by other methods.

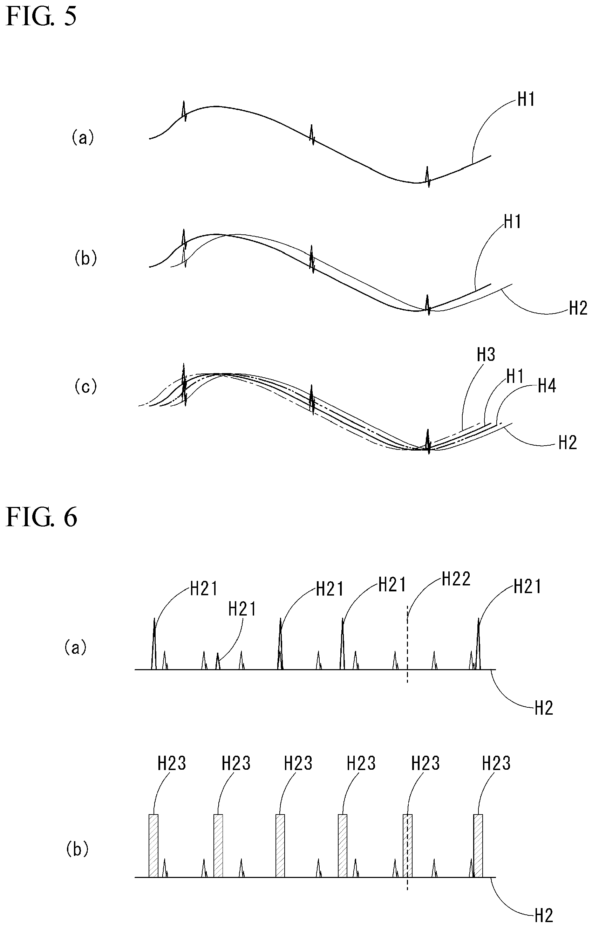

For example, FIG. 5(a) illustrates waveform data H1 indicating a portion of the real part ("real" illustrated in FIG. 4) of the separation signal. When the Hilbert transform is performed on the real part of the separation signal, waveform data H2 of the imaginary part of the separation signal is generated as illustrated in FIG. 5(b). Then, waveform data H3 and waveform data H4 including the imaginary part signal of the separation signal are combined. When the waveform data items H1 to H4 are combined (waveform combination), the result is as illustrated in FIG. 5(c).

In each of the waveform data items H1 to H4, both a high-frequency component and a low-frequency component deviate by 1/4 cycle (= 90°) as illustrated in FIG. 5(c) (for example, the shape and height of the peak of the waveform data change). However, since the low-frequency component has a longer cycle than the high-frequency component, the deviation of the low-frequency component is large. That is, the peak position of the high-frequency component (an R-wave component of the fetal electrocardiographic component) changes little. Therefore, the signal of the peak position can be easily extracted by the following process of the specific frequency band component extraction unit 512.

### [Configuration of Specific Frequency Band Component Extraction Unit]

The specific frequency band component extraction unit 512 extracts specific frequency band components of four complex signals for each of the channels D1 to D11. Since the R-wave component of the fetal electrocardiographic component includes many frequencies corresponding to 10 Hz to 40 Hz, a frequency band component of, for example, 10 to 40 Hz is extracted. When the process of the specific frequency band component extraction unit 512 is performed, a high-frequency component (40 Hz) remains and a low-frequency component becomes flat. At this time, the high-frequency components of each complex signal may have slightly different frequency levels depending on the angle (for example, "real" or "image"). However, a high-frequency component is extracted from any complex signal.

### [Configuration of Extraction Unit]

The extraction unit 513 converts the specific frequency band component extracted by the specific frequency band component extraction unit 512 into an absolute value, calculates the standard deviation σ, standardizes the specific frequency band component, and extracts the fetal feature display signal. Specifically, the extraction unit 513 calculates the standard deviation σ of the specific frequency band component and performs division by the standard deviation σ to uniformize the noise levels of each channel. Therefore, only the comparison of the peak heights makes it possible to easily determine which channel peak is more conspicuous than noise.

Here, the specific frequency band component includes noise, and 95% of the noise falls within a range of ± 2σ. Therefore, less than 2.5σ is regarded as a base range. Further, the R-wave component (peak value) of the fetal component is within a range of 3σ to 5σ, and a range exceeding 10σ is likely to be a large amount of noise. Therefore, after calculating the standard deviation σ, the extraction unit 513 performs division by the standard deviation σ and extracts 2.5σ to 10σ as the fetal feature display signal. The fetal feature display signal obtained by removing most of noise from the specific frequency band component is generated by the process of the extraction unit 513.

In the above-described embodiment, the extraction unit 513 extracts 2.5σ to 10σ as the fetal feature display signal. However, the invention is not limited thereto. This range may be appropriately set.

### [Configuration of Maternal Electrocardiographic Signal Removal Unit]

The maternal electrocardiographic signal removal unit 52 removes a fetal feature display signal at the timing when the electrocardiographic signal of the mother M is likely to appear from the fetal feature display signals to obtain a fetal feature signal including a large number of electrocardiographic signals of the fetus M1. The timing when the electrocardiographic signal of the mother M is likely to appear is the cycle (the cycle indicated by a large waveform H21 and a dashed line H22 in FIG. 6(a)) of the electrocardiographic signal of the mother M that has been detected in advance from the biological signal acquired from the maternal electrode 2B by the fetal component extraction processing unit 5.

The maternal electrocardiographic signal removal unit 52 sets the fetal feature display signal at the timing when the electrocardiographic signal of the mother M is likely to appear (for example, 50 samples before and after the timing among 15000 samples) to "0" in order to make the fetal electrocardiographic component conspicuous in the data H2 of the fetal feature display signal (see FIG. 6(a)). Therefore, a signal in a range H23 surrounded by a dashed line in FIG. 6(b) is set to "0" (removed), and the data H2 in which the fetal electrocardiographic component is conspicuous is generated.

Therefore, even in a case in which the electrocardiographic signal of the mother M remains in the fetal feature display signal, the fetal feature display signals before and after the timing when the electrocardiographic signal of the mother M is likely to appear are removed to obtain a fetal feature signal in which the fetal electrocardiographic component is conspicuous.

The fetal electrocardiographic component and the electrocardiographic signal of the mother M are likely to overlap each other. However, the fetal electrocardiographic component is removed together with the electrocardiographic signal of the mother M to be "0". Further, even in a case in which the electrocardiographic signal of the mother M does not appear, the fetal feature display signals before and after the timing when the electrocardiographic signal of the mother M is likely to appear (for example, the timing represented by the dashed line H22) are removed to be "0". That is, the fetal feature display signals before and after the timing when the electrocardiographic signal of the mother M is likely to appear are set to "0" without determining whether or not the electrocardiographic signal of the mother M appears.

### [Configuration of Data Compression Unit]

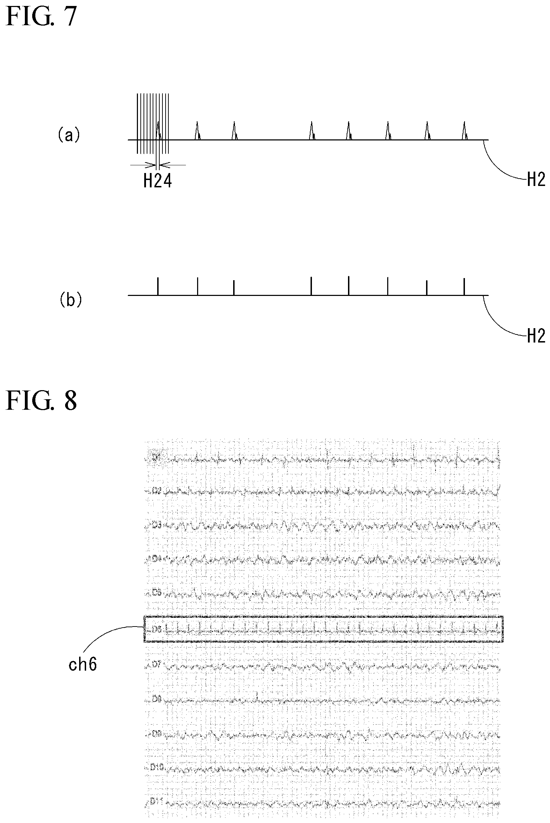

The data compression unit 53 compresses each set of a plurality of samples of the fetal feature signal for each channel to generate compressed data. Specifically, 15000 samples for each channel are compressed to 300 samples. At this time, the maximum value of every 50 samples (a range H24 surrounded by each line in FIG. 7(a)) is extracted, and the 50 samples are set as one sample including the maximum value. Data of adjacent samples among the obtained 300 samples (× 44 channels) are compared with each other and compressed data in which data of samples other than the sample with the largest maximum value is set to "0" is newly created.

Here, the maximum value of the R-wave of the fetal electrocardiographic component is about 240 bpm, and it is highly likely that noise is detected at 400 bpm or more. Therefore, even in a case in which the R-wave of the fetal electrocardiographic component is 400 bpm, one or more R-wave components of the fetal electrocardiographic component are not included in 150 samples. Therefore, in a case in which the maximum value of one sample (50 samples before compression) of the data of 300 samples after compression is smaller than the maximum values of adjacent samples on both sides of the one sample, it is treated as "0", and compressed data illustrated in FIG. 7(b) in which the R-wave component of the fetal electrocardiographic component is included in one of every three samples is generated.

### [Configuration of Signal Selection Unit]

The signal selection unit 54 calculates the standard deviation of the compressed data of each channel. In a case in which the R-wave component of the fetal electrocardiographic component can be detected in the compressed data, many peak values can be detected and the value of the standard deviation is large. Therefore, the compressed data is sorted in descending order of standard deviation for each channel, and compressed data having the second largest standard deviation is selected. Then, the selected compressed data of each channel is sorted in descending order of the standard deviation.

The reason why the compressed data with the second largest standard deviation is selected is that, when the compressed data with the largest standard deviation is selected, sudden noise is included. Therefore, the compressed data with the largest standard deviation is not selected.

Specifically, the complex signal generation unit 511 generates four complex signals. However, since the signals are originally the same data (the signals separated by the independent component analysis with reference), the standard deviations are substantially the same. Data with a large standard deviation due to sudden noise is removed. Among the remaining data items having substantially the same standard deviation, the compressed data with the largest standard deviation is selected.

The signal selection unit 54 selects the compressed data having the second largest standard deviation. However, the invention is not limited thereto. For example, the signal selection unit 54 may select the compressed data having the largest standard deviation or may select the compressed data having the third or fourth largest standard deviation.

### [Configuration of Image Generation Unit]

The image generation unit 55 sorts the separation signals corresponding to the compressed data having the second largest standard deviation in descending order of the standard deviation and displays them on the monitor 7. Specifically, the image generation unit 55 generates an image in which the separation signals of each channel are re-sorted in descending order of the number of fetal electrocardiographic components and outputs the image to the monitor 7.

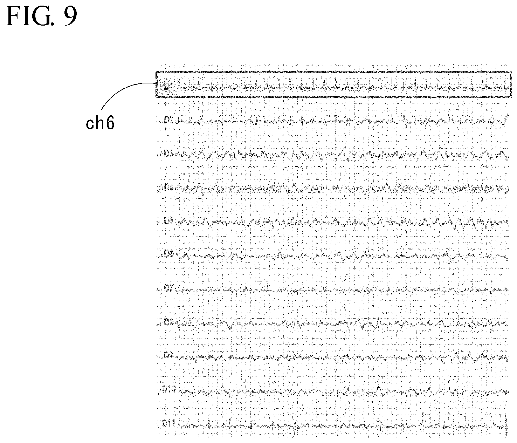

For example, in an image (see FIG. 8) in which the waveform data (signals corresponding to fetal electrocardiograms) of the separation signals separated by the fetal component extraction processing unit 5 is displayed in the order of channels, it is difficult to know which signal is the separation signal including a large number of fetal electrocardiographic components. However, in this embodiment, in the image generated by the image generation unit 55, the separation signal including a large number of fetal electrocardiographic components is disposed on the uppermost side (see FIG. 9).

Specifically, in FIG. 8, the data items D1 to D12 based on the separation signals of each channel are displayed in the order associated with the channels. The data of a channel ch6 including a large number of fetal electrocardiographic components is displayed sixth from the top. However, in FIG. 9, the data of the channel ch6 including a large number of fetal electrocardiographic components is displayed first from the top. The image illustrated in FIG. 9 is displayed on the monitor 7, which makes it possible for the medical staff to reliably select the waveform data (a signal corresponding to the fetal electrocardiogram) of the separation signal including a large number of fetal electrocardiographic components.

### [Fetal Electrocardiographic Signal Processing Method]

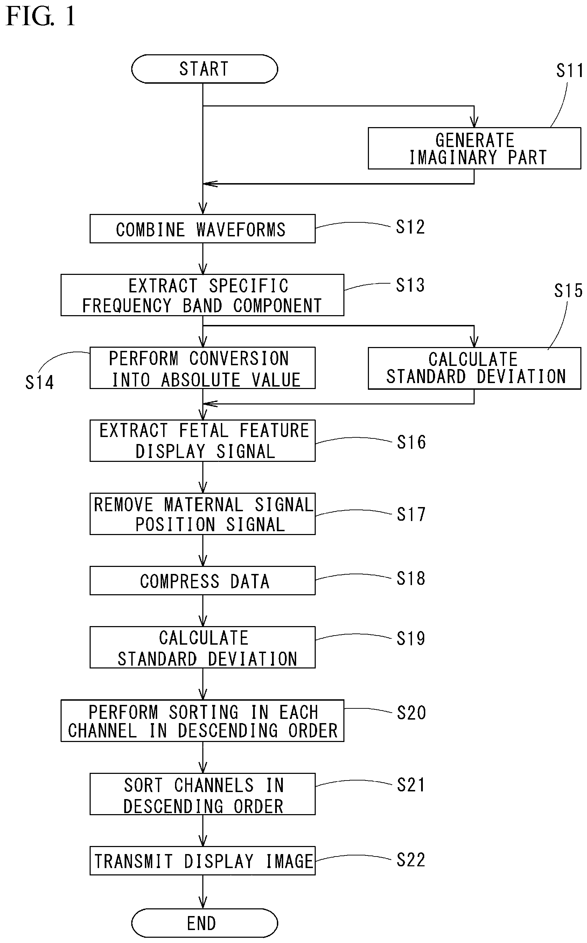

The channel re-sorting unit 6 (fetal electrocardiographic processing device) described above performs fetal electrocardiographic processing according to the procedure illustrated in FIG. 1.

First, the complex signal generation unit 511 performs the Hilbert transform on the separation signals of each of the channels D1 to D11 to generate the imaginary parts (image illustrated in FIG. 4) of the separation signals (Step S11). Then, the complex signal generation unit 511 performs waveform combination to generate four complex signals including the imaginary part signal generated in Step S11 for each of the channels D1 to D11 (Step S12).

After Step S12, the specific frequency band component extraction unit 512 extracts the specific frequency band components of the four complex signals for each of the channels D1 to D11 (Step S13). Then, the extraction unit 513 converts the specific frequency band component extracted in Step S13 into an absolute value (Step S14) and calculates the standard deviation σ (Step S15). Then, after calculating the standard deviation σ, the extraction unit 513 performs division by the standard deviation σ and extracts 2.5σ to 10σ as the fetal feature display signal (Step S16).

Therefore, the fetal feature display signal in which most of the noise has been removed from the specific frequency band component is extracted. That is, the process in Steps S11 to S16 corresponds to a fetal feature display signal extraction step of the invention, Steps S11 and S12 correspond to a complex signal generation step, and Step S13 corresponds to a specific frequency band component extraction step.

After the noise removal step (Steps S11 to S16) is performed, the maternal electrocardiographic signal removal unit 52 performs a maternal electrocardiographic signal removal step that removes a fetal feature display signal at the timing when the electrocardiographic signal of the mother M is likely to appear from the standardized specific frequency band components to obtain a fetal feature signal including a large number of electrocardiographic signals of the fetus M1 (Step S17).

Then, the data compression unit 53 performs a data compression step that allocates a plurality of samples forming the fetal feature signal of each channel in units of a predetermined number of samples, extracts the maximum value of the predetermined number of allocated samples, and sets the predetermined number of samples as one sample including the maximum value to generate compressed data (Step S 18).

After Step S18 is performed, the signal selection unit 54 calculates the standard deviation of the compressed data of each channel (Step S19), sorts the compressed data for each channel in descending order of the standard deviation (Step S20), selects compressed data (a separation signal corresponding to the compressed data) with the second largest standard deviation among the channels, and sorts the compressed data in descending order of the standard deviation (Step S21). The process in Steps S18 to S21 corresponds to a signal selection step of the invention.

Then, the image generation unit 55 generates an image in which the separation signals of each channel are re-sorted in descending order of the number of fetal electrocardiographic components (descending order of the standard deviation) and outputs the image to the monitor 7 (Step S22). Then, the channel re-sorting process (fetal electrocardiographic signal processing) ends.

In this embodiment, noise is removed from the separation signals to extract the fetal feature display signals and a signal at the timing when the electrocardiographic signal of the mother M is likely to be included is removed from the extracted fetal feature display signals. Therefore, it is possible to reliably remove noise and the electrocardiographic signal of the mother M. This configuration makes it possible to obtain a fetal feature signal including a large number of electrocardiographic signals of the fetus M1. The fetal feature signals of each channel can be compared to appropriately select a separation signal (fetal electrocardiographic signal) that reliably includes a fetal electrocardiographic component among the separation signals.

Further, in this embodiment, the phase of the separation signal is shifted for each channel to generate a plurality of complex signals including an imaginary part signal, which makes it possible to increase the number of samples for extracting the fetal feature signal and to obtain a plurality of fetal feature signals for each channel. Therefore, since the number of fetal feature signals to be compared is large, it is possible to appropriately select a separation signal (fetal electrocardiographic signal) that reliably includes the fetal electrocardiographic component from the separation signals.

Furthermore, in this embodiment, it is possible to select a separation signal including a large number of fetal electrocardiographic components, using the compressed data generated by allocating a plurality of samples forming the fetal feature signal in units of a predetermined number of samples, extracting the maximum value of the predetermined number of allocated samples, and setting the predetermined number of samples as one sample including the maximum value. Therefore, it is possible to reduce the processing time. Further, a separation signal of a channel corresponding to the compressed data with the largest standard deviation is selected, which makes it possible to more reliably select a separation signal including a large number of fetal electrocardiographic components.

As such, it is possible to display a signal corresponding to a more accurate fetal electrocardiogram on the monitor 7 in real time. Therefore, the probability of accurately selecting a channel including many features of the fetal electrocardiographic component from the analyzed and extracted signals of each channel increases. As a result, when waveform data is displayed on the monitor 7, it is possible to check a signal including many features of the fetal electrocardiographic component (a signal corresponding to the fetal electrocardiogram), which helps determination during measurement. In addition, it is possible to reduce the time and effort required for double check after measurement.

Further, the possibility of selecting a channel including many features of the fetal electrocardiographic component increases. Therefore, the possibility of recognizing the position of the R-wave for each heartbeat increases and this is reanalyzed as a reference or a prediction model to improve the accuracy of detecting the peak of the R-wave and to more accurately estimate an electrocardiographic component. In addition, it is possible to accurately calculate the fetal heart rate for each heartbeat.

Further, the invention is not limited to the above-described embodiment and various modifications and changes of the invention can be made without departing from the scope and spirit of the invention.

### Industrial Applicability

It is possible to appropriately select a signal that reliably includes a fetal electrocardiographic component from a plurality of signals separated by the independent component analysis with reference.

### Reference Signs List

1: medical apparatus (fetal electrocardiographic signal processing device)
2A: electrode (fetal electrode)
2B: electrode (maternal electrode)
3: apparatus main body
4: preprocessing unit
5: fetal component extraction processing unit
6: channel re-sorting unit
7: monitor
51: fetal feature display signal extraction unit
52: maternal electrocardiographic signal removal unit
53: data compression unit
54: signal selection unit
55: image generation unit
511: complex signal generation unit
512: specific frequency band component extraction unit
513: extraction unit
M: mother
M1: fetus

## Claims

1. A fetal electrocardiographic signal processing method comprising:
a fetal feature display signal extraction step of separating a separation signal for each channel from biological signals of a plurality of channels acquired from a pregnant mother, using independent component analysis with reference, and removing noise from each separation signal to extract a fetal feature display signal for each channel; and
a maternal electrocardiographic signal removal step of removing the fetal feature display signal at a timing when an electrocardiographic signal of the mother is likely to appear from the fetal feature display signals to obtain fetal feature signals including a large number of fetal electrocardiographic signals.

2. The fetal electrocardiographic signal processing method according to claim 1,
wherein the fetal feature display signal extraction step includes:
a complex signal generation step of generating a plurality of complex signals including an imaginary part signal obtained by shifting a phase of the separation signal for each channel; and
a specific frequency band component extraction step of extracting a specific frequency band component of each complex signal for each channel, and
the fetal feature display signal obtained by removing the noise from the specific frequency band component is acquired.

3. The fetal electrocardiographic signal processing method according to claim 2, further comprising:
a data compression step of compressing a plurality of samples forming the fetal feature signal to generate compressed data for each channel;
a signal selection step of calculating a standard deviation of the compressed data based on each complex signal for each channel, selecting compressed data with a second largest standard deviation, and sorting the selected compressed data of each channel in descending order of the standard deviation; and
an image generation step of sorting and displaying the separation signals corresponding to the compressed data sorted in descending order of the standard deviation,
wherein, in the data compression step, the plurality of samples are allocated in units of a predetermined number of samples, a maximum value of the predetermined number of allocated samples is extracted, and the predetermined number of samples are set as one sample including the maximum value.

4. A fetal electrocardiographic signal processing device comprising:
a fetal feature display signal extraction unit that separates a separation signal for each channel from biological signals of a plurality of channels acquired from a pregnant mother, using independent component analysis with reference, and removes noise from each separation signal to extract a fetal feature display signal for each channel; and
a maternal electrocardiographic signal removal unit that removes the fetal feature display signal at a timing when an electrocardiographic signal of the mother is likely to appear from the fetal feature display signals to obtain fetal feature signals including a large number of fetal electrocardiographic signals.

5. The fetal electrocardiographic signal processing device according to claim 4,
wherein the fetal feature display signal extraction unit includes:
a complex signal generation unit that generates a plurality of complex signals including an imaginary part signal obtained by shifting a phase of the separation signal for each channel; and
a specific frequency band component extraction unit that extracts a specific frequency band component of each complex signal for each channel, and
the fetal feature display signal extraction unit acquires the fetal feature display signal obtained by removing the noise from the specific frequency band component.

6. The fetal electrocardiographic signal processing device according to claim 5, further comprising:
a data compression unit that compresses a plurality of samples forming the fetal feature signal to generate compressed data for each channel;
a signal selection unit that calculates a standard deviation of the compressed data based on each complex signal for each channel, selects compressed data with a second largest standard deviation, and sorts the selected compressed data of each channel in descending order of the standard deviation; and
an image generation unit that sorts and displays the separation signals corresponding to the compressed data sorted in descending order of the standard deviation,
wherein the data compression unit allocates the plurality of samples in units of a predetermined number of samples, extracts a maximum value of the predetermined number of allocated samples, and sets the predetermined number of samples as one sample including the maximum value.
